# EUROPEAN PATENT SPECIFICATION

(11) **EP 4 247 299 B1**
(45) Date of publication and mention of the grant of the patent: **07.05.2025**
(21) Application number: 21894157.3
(22) Date of filing: 18.11.2021
(51) Int. Cl.: A61F 2/16, H03J 3/24, H03J 9/02

(54) **SYSTEM PROVIDING POWER AND COMMUNICATIONS FOR OCULAR DEVICE**
SYSTEM ZUR BEREITSTELLUNG VON STROM UND KOMMUNIKATION FÜR EINE OKULARVORRICHTUNG
SYSTÈME FOURNISSANT DE L'ÉNERGIE ET DES COMMUNICATIONS POUR DISPOSITIF OCULAIRE

(30) Priority: 18.11.2020 US 202063115064 P
(43) Date of publication of application: 27.09.2023
(73) Proprietor: Blink Energy Ltd., Haifa (IL)
(72) Inventor: COHEN, Nadav, 3082500 Ein Ayala (IL); BAR-ON, Yariv, Yavne (IL); DAFAN, Roni, 9322612 Jerusalem (IL)
(74) Representative: Kancelaria Eupatent.pl Sp. z.o.o
(86) International application number: PCT/IB2021/060707
(87) International publication number: WO 2022/107043

(56) References cited:
- WO-A1-2019/220307
- US-A1- 2010 016 704
- US-A1- 2013 041 245
- US-A1- 2013 150 771
- US-A1- 2013 194 540
- US-A1- 2015 057 516
- US-A1- 2018 043 646
- US-A1- 2018 043 646
- US-A1- 2019 245 523
- US-A1- 2020 138 565

## Description

### FIELD AND BACKGROUND OF THE INVENTION

The present invention relates to ocular devices and, in particular, it concerns a system in which an ocular device is powered by an externally-mounted device.

Smart ocular devices (electronic units in the vicinity of the eye) all need a power source and many of them need to transmit data to an external unit (hub, cellphone, cloud). In many cases there is little space for a significant energy storage limiting the energy available (e.g., minimal space on a smart contact lens). Use of a small power source limits the distance of the communication as well as shortening the operational time, and may preclude certain applications due to insufficient power.

Some conventional devices employ additional power supplied periodically (usually wirelessly) via a hand-held external device which is intermittently brought close to the eye so as to charge the ocular device and/or download data therefrom via some type of coupling. One such example, as disclosed in US2018043646A1, is a contact lens that transmits/receives data and power to and from an external unit which is periodically positioned adjacent to the contact lens.

### SUMMARY OF THE INVENTION

The present invention is a system in which an ocular device is powered by an externally-mounted device according to claims 1 and 3. Further embodiments are provided in the dependent claims.

### BRIEF DESCRIPTION OF THE DRAWINGS

The invention is herein described, by way of example only, with reference to the accompanying drawings, wherein:
FIG. 1A is a schematic front view of an external device, constructed and operative according to the teachings of an embodiment of the present invention, for providing power and/or communications for an ocular device, implemented as a self-contained eyelid-mounted unit;
FIG. 1B is a schematic front view of an ocular device, implemented as a smart contact lens, for use in a system with the external device of FIG. 1A;
FIGS. 2A-2D are schematic frontal illustrations of four exemplary options for deployment of the device of FIG. 1A in proximity to the eye of a user;
FIGS. 3A and 3B are isometric views illustrating deployment of a variant implementation of the system of the present invention in which the external device is subdivided into an eyelid-mounted unit and an additional unit;
FIGS. 4A and 4B are sagittal cross-sectional views through the eye illustrating a further variant implementation of the system of the present invention, where the ocular device is a prosthetic lens and the external device is mounted on an upper eyelid, the eye being shown in a closed state and an open state, respectively;
FIGS. 5 and 6 are isometric views similar to FIG. 3A illustrating alternative deployment locations for the additional unit at the user's temple and in an over-the-ear unit, respectively;
FIGS. 7A and 7B are graphs illustrating the time variation of eyelid angular displacement and angular velocity, respectively, during a blinking motion of the eye;
FIG. 8A is a block diagram of a system according to an embodiment of the present invention employing non-resonant inductive coupling between the external device and the ocular device;
FIG. 8B is a block diagram of a system according to a further embodiment of the present invention employing near-field resonant coupling between the external device and the ocular device;
FIG. 9A is a block diagram of a system according to a further embodiment of the present invention employing near-field resonant coupling for both power transmission and data transfer between the external device and the ocular device;
FIG. 9B is a schematic graph of RF oscillator output for transmission of data from the external device to the ocular device in the system of FIG. 9A;
FIG. 9C is a schematic graph of transmission resonant circuit current variations for conveying data encoded by load modulation at the ocular device in the system of FIG. 9A; and
FIG. 9D is a schematic graph illustrating selective operation of system transmission synchronized with blinking motions according to a further implementation of the present invention.

### DESCRIPTION OF THE PREFERRED EMBODIMENTS

The present invention is a system in which an ocular device is powered by an externally-mounted device, and corresponding methods.

The principles and operation of systems and methods according to the present invention may be better understood with reference to the drawings and the accompanying description.

By way of introduction, the present invention provides a system and corresponding methods in which an ocular device deployed in or on an eyeball is operated in combination with a device mounted externally on the user's body, where the external device provides power to, and/or data communication with, the ocular device. In order to implement such a system effectively, aspects of the present invention address various preferred options for the location and manner of deployment of the external device in order to achieve sufficient proximity to the ocular device, and various coupling schemes for achieving highly efficient energy transfer and/or communications between the ocular device and the external device.

Provision of an external body-mounted device in proximity to an ocular device to provide energy and/or energy-efficient communication with the ocular device opens up a wide range of applications for which power requirements would otherwise be prohibitive or at least limiting for a stand-alone ocular device.

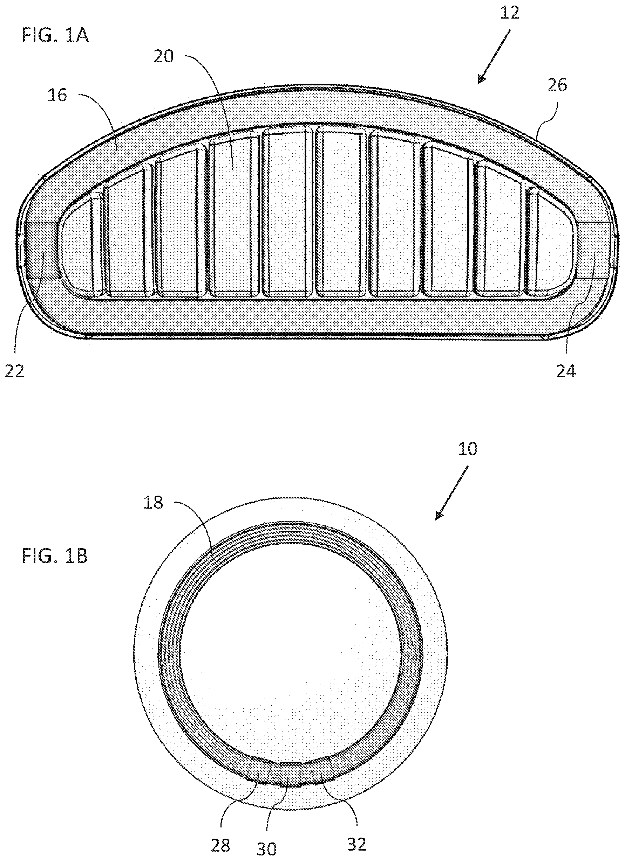

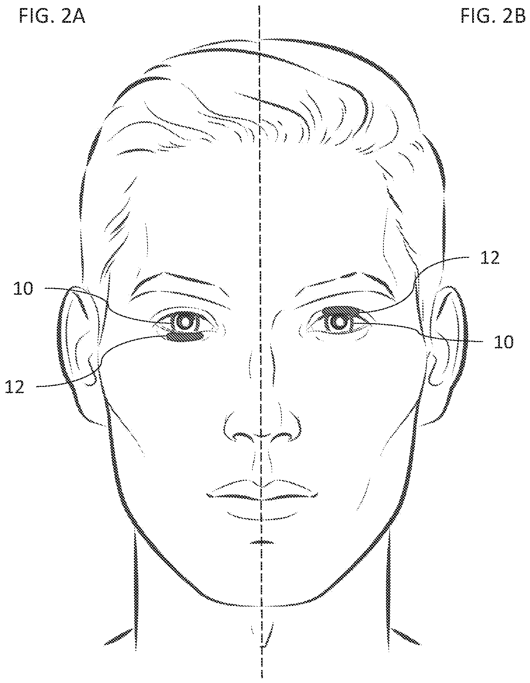

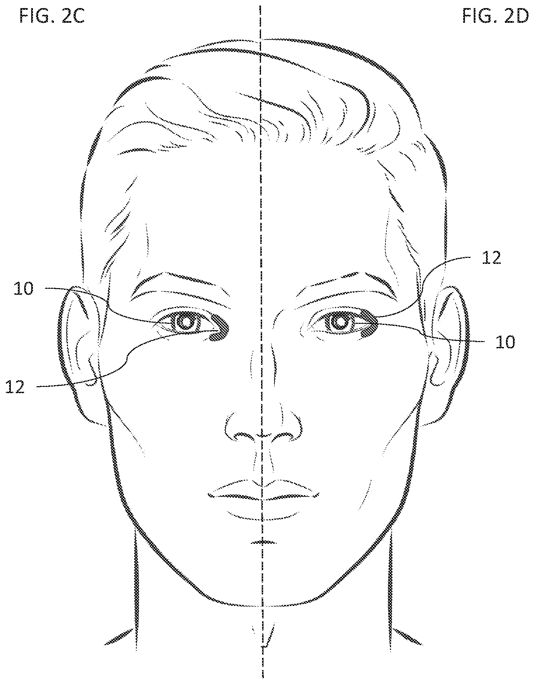

Referring now to the drawings, FIGS. 1A-6 illustrate various implementations options for components of a system, constructed and operative according to the teachings of certain embodiments of the present invention. In general terms, the system includes an ocular device **10** (e.g., FIG. 2), for deployment in or on an eyeball, and an external device **12** (e.g., FIG. 1). The ocular device is typically either an ocular implant or a contact lens (CL).

For the purpose of this document, unless otherwise stated or self-evident from the context, the term "ocular" in relation to positioning of a device refers to any location that is internal to the eyelid when closed, whether applied to an outer surface of the eye ("supra-ocular", e.g., a contact lens) or whether implanted within tissue of the eye ("intra-ocular"). Conversely, the term "extra-ocular" or simply "external" refers to any location that is outwards from at least part of a thickness of the eyelid, or otherwise positioned externally to the eye.

Implant examples include, but are not limited to, one or more of the following devices or functions: intra-ocular pressure (IOP) sensors, retinal prostheses, implants for minimally invasive glaucoma surgery (MIGS) with active or sensing components, glucose metering, drug delivery devices, accommodative/refractive lenses, dry-eye treatments, and tissue restoration.

Smart device examples include, but are not limited to, one or more of the following devices or functions: IOP contact lens (CL), drug delivery CL, accommodative/refractive CL, augmented reality CL, drug delivery CL, drug eye management CL, blinking stimulation CL, drug delivery patch, drug eye management patch, communication patch, blinking stimulation patch (for facial paralysis).

In each of the above cases, the components of the ocular device are generally standard, and will not be described herein, other than those required for the energy transfer and/or communication according to the teachings of the present invention, which are described below.

### Form Factor of External Device

At least part of external device **12** is configured for mounting in proximity to the eye, meaning within about 50 millimeters from the closest part of the eyeball, and more preferably within about 30 millimeters thereof. In certain preferred cases, the external device overlaps the orbital margin (edge of the eye socket), and in certain particularly preferred implementations, the at least part of the external device which is in proximity to the eye is located inwards from the orbital margin, and most preferably on an eyelid of the user. The device may be attached in the form of an adhesive patch, or may be deployed as an implant.

The term eyelid is used herein to refer generically to the upper eyelid, the lower eyelid, and the regions of eyelid tissue adjacent to the medial or lateral canthus, i.e., at the sides of the eye. Thus, FIGS. 2A-2D illustrate various particularly preferred options according to which external device **12** is mounted on the lower eyelid (FIG. 2A), the upper eyelid (FIG. 2B), around the medial canthus (FIG. 2C) and around the lateral canthus (FIG. 2D). In the case of lateral or medial positioning, the device may either be sufficiently flexible to accommodate the local motion of the eyelid tissue during blinking, or may be placed slightly further outwards, on the orbital margin, in a region which does not undergo significant motion during blinking.

Optionally, as illustrated in FIG. 1, the eyelid-mounted unit may include all required components, and operate as a stand-alone external device **12.** This is typically the preferred option for applications in which the required power storage and other design considerations allow it. In order to remain comfortable to the user, the overall weight and dimensions should remain sufficiently small that it does not impede normal function of the user. Thus, for an eyelid-mounted external device, weight is preferably limited to no more than 2 grams, and more preferably no more than 1 gram, while thickness is preferably limited to no more than 2 mm, and more preferably no more than 1.5 mm. The area may be upwards of 10 percent of the total area of the eyelid when closed, and in some cases may be 20-50 percent of that area, or more. In quantitative terms, the area of the eyelid-mounted unit for certain preferred implementations is in the range of 1-2 square centimeters.

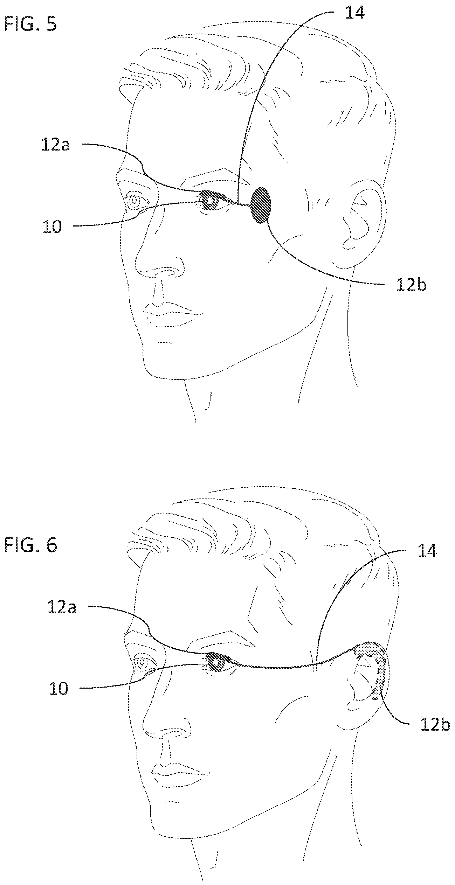

In other cases, the external device may be subdivided into an eyelid-mounted unit **12a** and an additional unit **12b** that is located nearby, preferably connected to the eyelid-mounted unit via a wired connection **14.** In the example of FIG. 5, additional unit **12b** is mounted via adhesive to the temple region of the user, while in the example of FIG. 6, it is mounted in an over-the-ear housing. Other locations can also be used. The provision of an additional unit **12b** mounted separately from the eyelid-mounted unit **12a** facilitates provision of much larger energy storage than can be incorporated on an eyelid-mounted device alone, and may also allow integration of other relatively large, heavy and/or energy consuming components that may be required for certain applications. In these cases, the eyelid-mounted unit may be minimized to include only the components for which eyelid positioning is beneficial, such as the transmitter antenna and an accelerometer (if present).

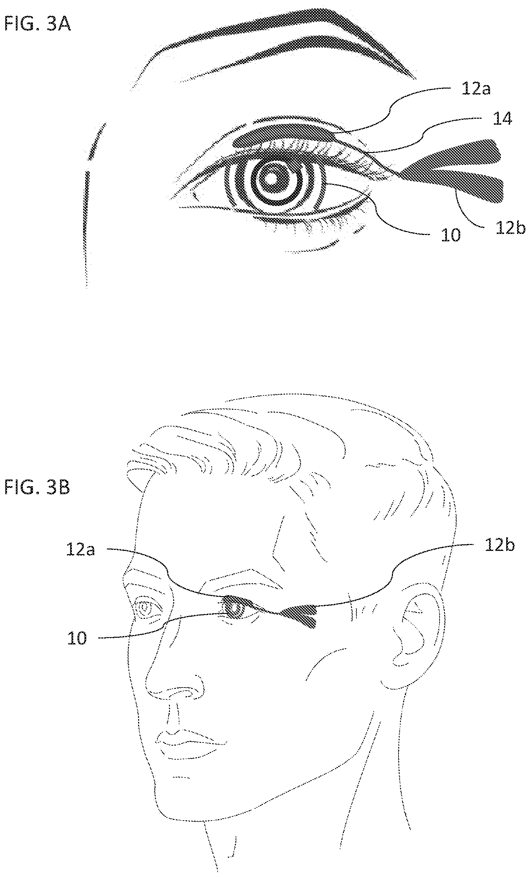

In some cases, comfort may be enhanced by implementing additional unit **12b** as a flexible unit, for example, with electronic components attached to a flexible substrate and formed into a patch with flexible encapsulation. Optionally, such a structure may be bifurcated or otherwise provided with slots or other features to enhance flexibility and relative mobility of the parts of the unit, thereby minimizing interference with motion of the underlying skin, such as during smiling. Such an example is illustrated schematically in FIGS. 3A and 3B.

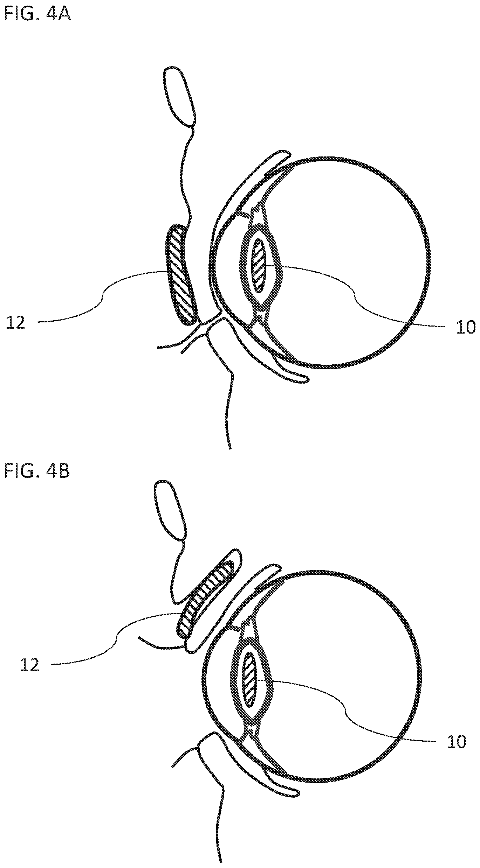

According to a further aspect of the present invention, the external device **12** (or unit **12a** thereof) is mounted on an outer surface of the user's upper eyelid without inhibiting a normal blinking motion of the eyelid. Although conventional thinking generally regards the external eyelid surface as unavailable for device mounting, since it folds into a greatly reduced volume when the eye is open, it has been found that the external eyelid surface can in fact be used to advantage to mount various device components, and according to one particularly preferred but non-limiting example, an electrically conductive loop or coil. Depending on the degree of flexibility of the eyelid-mounted device and its size, shape and positioning, the device on the eyelid may slightly modify the manner in which the eyelid tissue folds when the eyelid is open, and in some cases, part of the component may extend somewhat inwards between the eyeball and the tissue above the eye when the eyelid is open. This is illustrated schematically in FIGS. 4A and 4B, which show an eyelid-mounted external device **12** when the eyelid is closed (FIG. 4A, e.g., during blinking or sleep), and the position of the device when the eyelid is open, in a normal waking state (FIG. 4B). As seen in FIG. 4B, device **12** assumes a position at least partially enveloped in a pocket formed above the superior tarsus as the eyelid retracts.

Parenthetically, while most of the examples illustrated in the drawings show a smart contact lens as an example of the ocular device, FIGS. 4A and 4B illustrate an implant in the form of a smart ocular lens implant **10** implanted within the lens sack of the eye. The structural features and principles of operation taught by the present invention are fully equivalent for all types of applications, whether employing contact lenses or implants, other than the application-specific features of the contact lens or implant itself, which are not part of the invention per se. All features described herein in the context of a contact lens example should be considered equally applicable to implants, and vice versa, except where explicitly stated otherwise.

In the case of a two-part implementation including a unit **12a** attached to the upper eyelid and an additional unit **12b** mounted separately, wired connection **14** should be sufficiently flexible and sufficiently long to accommodate the range of motion of the eyelid during closing and opening of the eye. It has been found, for example, that a relatively fine wired connection extending laterally from unit **12a** towards the lateral canthus or nearby does not impact the comfort of eye movements and blinking in any noticeable way. Other locations, such as just above the eye, or any other location which is relatively close to the eye while not being displaced significantly during blinking.

Implementations in which the transmitting antenna is located on the upper eyelid have distinctive features compared to other mounting positions in which less motion typically occurs. On one hand, in the closed position of the eye (FIG. 4A), for certain antenna designs, an optimal degree of overlap and proximity is achieved between the transmitting antenna and the receiving antenna, at least for centrally positioned contact lenses or implants, presenting an opportunity for highly effective coupling for energy transfer and/or data communication. On the other hand, when the subject is awake, this state of maximal overlap occurs only briefly during blinking, and the degree of coupling between the transmitter antenna and the receiver antenna may vary significantly. These changes in coupling can be detected by the transmitter circuitry as a variation in the impedance load of the transmitter circuit, allowing for detection and analysis of a blinking motion. Additionally, or alternatively, blinking motion can be directly sensed by including an accelerometer (typically a gyroscopic MEMS accelerometer) either integrated into the eyelid-mounted device or mounted alongside.

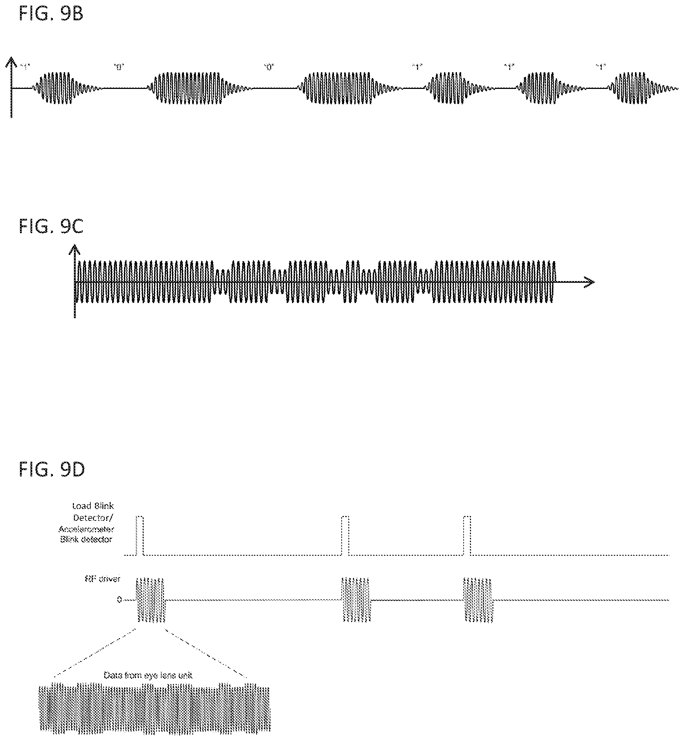

In certain subsets of applications of the present invention as further exemplified below, it may be advantageous to operate the device synchronously with at least part of a blinking motion cycle of the eye of the user. One such implementation will be discussed further below, with reference to FIG. 9D. It should be noted however that the present invention is not limited to such implementations, and that in some applications, operation of the device may occur continuously, or at intervals, without being triggered by any specific eyelid motion.

Where continuous or intermittent supply of power to the ocular device is required without being limited to synchronization with blinking, sensing of the variations in coupling can be used to provide an estimate of the power transfer efficiency, and thereby estimate the required power that must be delivered to the transmitter antenna in order to generate sufficient power at the ocular device. The accuracy of this feedback can be further enhanced by data transfer from the ocular device back to the external device (detailed below) which conveys the level of power actually received by the ocular device, allowing closed-loop feedback to adjust the transmitted power level.

Sensing of the blink motion timing, derived from the coupling variations and/or from an accelerometer, can provide valuable data for blink analysis which can be used in various medical and non-medical contexts, such as for diagnosis of dry eye disease (DED), as well as providing inputs used for identifying drowsiness and other behavior-related variations in blink patterns that can be used in various contexts.

### Energy Coupling

In order to allow energy supply to ocular device **10,** the ocular device is implemented with an energy receiver, and external device **12** includes an electrically-actuated energy-transmitting component, preferably mounted on the eyelid of a user. External device also includes driver circuitry, electrically connected to the energy-transmitting component, for actuating the energy-transmitting component to transfer energy wirelessly to the energy receiver of the ocular device. The driver circuitry may either be integrated with the eyelid-mounted component, or can be partially or entirely located in additional component **12b,** where used.

The close proximity of the energy-transmitting component to the energy receiver allows effective transfer of energy using a range of techniques, including but not limited to energy transmission via far field or near field excitation at a wide range of frequencies, and in the near field, by inductive coupling or capacitive coupling. Most preferably, for near field coupling, in order to avoid losses due to power radiating to the far field, frequencies in the RF range are preferred, corresponding to wavelengths significantly greater than the antenna dimensions. In certain cases, energy transfer via other mechanisms, such as mechanical vibration (ultrasound) or optical transmission, may also be viable.

Although all such options fall within the broad scope of certain embodiments of the present invention, it has been found particularly effective to perform energy transfer from the external device to the ocular device by near-field resonant coupling. To this end, the energy receiver of ocular device **10** preferably includes a receiver resonant circuit including a near-field receiver antenna, and the energy-transmitting component of external device **12** preferably includes a near-field transmitter antenna which is part of a transmitter resonant circuit. The two resonant circuits are designed (tuned) at the same resonant frequency, and the driver circuitry drives oscillation of the transmitter resonant circuit at a tuned resonant frequency of the coupled resonant system so as to induce resonance in both the transmitter resonant circuit and the receiver resonant circuit. Power can then be recovered efficiently from the transmitter resonant circuit by the ocular device.

The use of resonant coupling in the reactive near-field is highly advantageous for maximizing energy transfer efficiency. By employing near-field antennae (typically of small dimensions relative to the wavelength), transmitted energy losses to the environment are kept to a minimum. The use of coupled resonant circuits in the transmitter and receiver ensures that energy losses of the driving circuitry are kept to a minimum, allowing relatively high power transfer efficiency even when coupling between the transmitter and receiver resonant circuits is relatively low.

The type of antenna responsible for the coupling between the two resonant circuits may be either the inductive component of the resonant circuits, i.e., an inductive antenna, or the capacitive component of the resonant circuit, i.e., a capacitive antenna. In certain particularly preferred implementations, such as illustrated in FIGS. 1A and 1B, the near-field transmitter antenna of external device **12** is an inductive antenna in the form of a coil **16,** and the near-field receiver antenna of ocular device **10** is an inductive antenna in the form of a coil **18.** The term "inductive near-field resonant coupling" is used herein synonymously with the term "magnetic resonance coupling."

Other components of external device **12** as illustrated in FIG. 1 typically include a battery **20,** an application-specific integrated circuit (ASIC) or other microcontroller unit (MCU) **22,** and other components such as low-energy Bluetooth (BLE) communication components, sensors and/or other peripheral electronic components **24,** all preferably encapsulated in a protective encapsulation **26.**

Other components of ocular device **10** as illustrated in FIG. 2 typically include an application-specific integrated circuit (ASIC) or other microcontroller unit (MCU) **28,** sensors **30** and other application-specific payload **32,** depending on the nature of the device. In the non-limiting example illustrated in FIG. 2, these elements are integrated into a soft contact lens, for deployment in contact with the cornea. The principles of operation remain the same for intraocular devices.

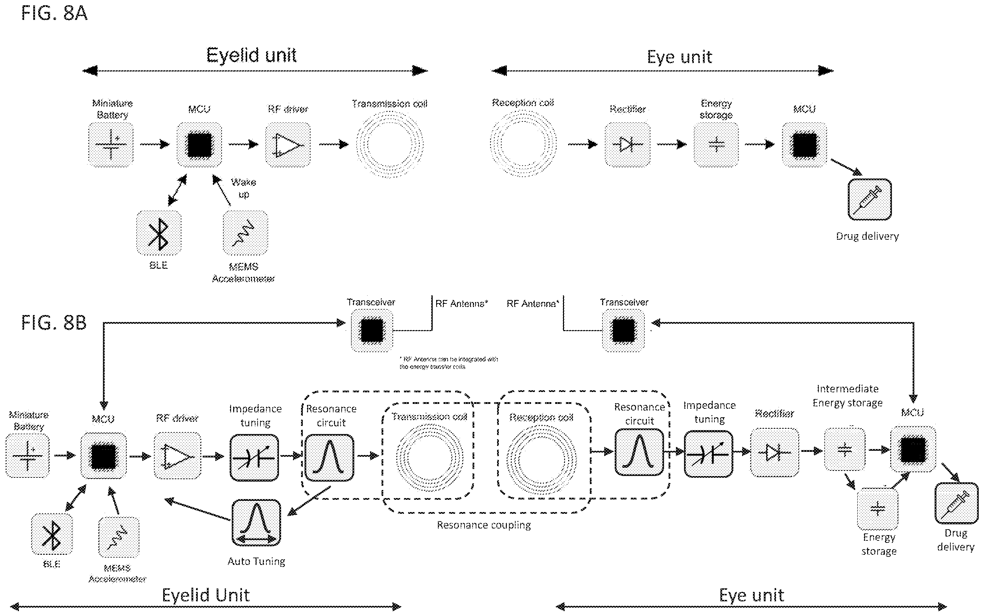

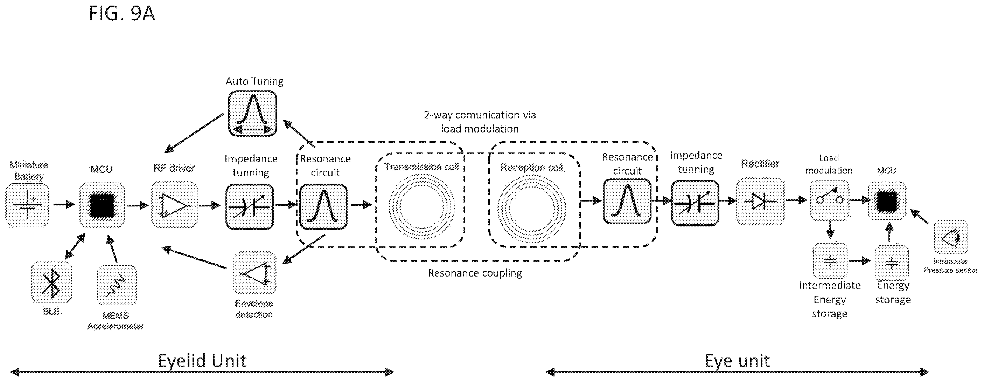

Further details of the typical components and functional relationships between them for various implementations of ocular device **10** and external device **12** will be described below, with reference to FIGS. 8A, 8B and 9A.

Particularly, but not exclusively, for external devices mounted on the upper eyelid, relative positioning of the transmitter antenna and the receiver antenna, and the resulting coupling therebetween, will vary considerably during operation, for example, due to blinking. The system preferably includes a resonance tracking (auto-tuning) circuit, which can detect and correct for resonance frequency shift arising from varying impedance due to relative movement between the two coils arising from the eye movement, and from additional parameter shifts such as temperature etc. This decreases the sensitivity for alignment and distance variations, facilitating maintenance of high efficiency and a constant supply of energy despite variations in position and other varying parameters. Resonance tracking circuits are known per se in the art, and can readily be implemented by a person ordinarily skilled in the art.

Optionally, where data transfer between the ocular device and the external device is available (as will be discussed below), the ocular device may transmit back to the external device information indicating the actual power received, thereby indicating the power transfer efficiency (PTE). This allows closed-loop operation in which the required power is transmitted on the basis of the power actually received.

### High-Power Applications

One aspect of the significance of providing power from an external device can be appreciated by referring to examples of high-power applications. High power applications, such as an augmented reality contact lens or various active prosthetics, require a lot of energy relative to what can be stored by an onboard battery within a contact lens. In some cases, even of all the available area within a contact lens is used for power storage, the power would only be sufficient for a few hours' operation or less.

Furthermore, inclusion of significant battery storage capacity is typically incompatible with use of a soft contact lens because the flexibility is lost, and the thickness of the contact lens quickly increases to make it uncomfortable. Inclusion of batteries into a soft contact lens is also typically incompatible with normal manufacturing techniques for soft lenses.

By moving the power source offboard to the external device, it becomes possible to implement even a high-power application using a thin and comfortable solution within a soft contact lens with hundreds of times more power than what can be packed within a contact lens.

By way of a numerical example, the maximum power that can be currently packed on to a contact lens is of an order of P=0.5mWh. This is based on an assumed energy density of 100Wh/liter. Thus, and application which requires roughly 0.5 mW of power can only operate for up to about 1 hour based on an onboard power supply.

In contrast, if using an external device that is a self-contained eyelid-mounted unit, the eyelid-mounted unit can carry a battery that supplies P=50mWh. Even if we assume an energy transfer efficiency of 20%, this would still make available P=10mWh, which would be sufficient for 20 hours of operation at 0.5 mW. For embodiments with an additional unit **12b,** the available power can be increased by tens of times beyond this figure.

Depending on the implementation, the power supplied from external device **12** can be provided continuously to ocular device **10,** where it is rectified and managed continuously to power the device. Alternatively, ocular device **10** may include a power storage element, such as a power storage capacitor, which allows continuous operation of the ocular device **10** if power from external device **12** is interrupted.

### Data Transmission

As an alternative or addition to the power transfer discussed thus far, according to a further aspect of the present invention, external device **12** is configured to transfer data to and/or from the ocular device. Here too, the presence of a body-mounted external device in proximity to the ocular device greatly reduces the power requirements for data transfer compared to direct communication from an ocular device to a more remote device. The external device **12** preferably also includes a wireless communications interface for communicating with a communications network or with a mobile electronic device, thereby allowing it to exchange data with an external device.

In basic terms, this aspect of the present invention employs a first communication subsystem that is part of the ocular device **10,** and the external device **12** has a second communication subsystem, preferably associated with antenna **16,** for transferring data wirelessly to and/or from the first communication subsystem.

In a particularly preferred subset of implementations, where external device **12** also provides power to ocular device **10,** this data communication is implemented via modulation of the power transmission to convey encoded data. For data transfer from the external device to the ocular device, driver circuitry of the external device is configured to convey data via modulation of an amplitude of power transfer to the ocular device, either as switching between two or more amplitude levels or by introducing brief interruptions in the transmitted power. The ocular device is implemented with corresponding electronic circuitry associated with the receiver resonant circuit and configured to derive the data from the modulation.

For data transfer from the ocular device **10** to the external device **12,** the ocular device includes electronic circuitry associated with the receiver resonant circuit and configured to convey data via load modulation of the receiver resonant circuit. This load modulation causes a corresponding variation in the current of the transmitter resonant circuit, which is detected and decoded by the circuitry of the external device **12.**

The ability to transfer information from the ocular device to the external device also facilitates optimized operation of power transfer to the ocular device. The ocular device may advantageously report back to the external device the actual power received by the ocular device, allowing the external device to adjust the transmitted power to reach a specific required power transfer target for operation of the ocular device.

Turning now to FIG. 8A, this illustrates a typical implementation of a system according to an embodiment of the present invention including an ocular device **10,** here exemplified by an eye unit for drug delivery, and an external device **12,** here exemplified by an eyelid-mounted unit. This example employs non-resonant inductive coupling.

Referring first to the eyelid unit, a microcontroller unit is powered by a miniature onboard battery and operates an RF driver to drive the transmission coil (antenna), thereby generating an oscillating electric and magnetic field which induces oscillating current in the receiver coil (antenna) of the ocular device. This current passes through a rectifier to an energy storage device (typically a capacitor), which powers the ocular device MCU which controls the device payload, in this non-limiting case a drug delivery mechanism.

Optionally, particularly where it is desired to operate the device intermittently, synchronously with blinking, a MEMS accelerometer may be provided in the eyelid unit to trigger operation of the MCU and RF transmission when a blinking motion is detected.

Optionally, a wireless communications subsystem, such as a Bluetooth low-energy (BLE) component may be incorporated into the eyelid unit to allow connection to an external device, such as for reporting the status of the battery and/or receiving scheduling data for when, and for how long, the ocular unit should be activated.

FIG. 8B illustrates a further example which is essentially similar to that of FIG. 8A, but here employs near-field resonant coupling (magnetic resonance coupling). In this case, the transmitter antenna (coil) and the receiver antenna (coil) are each implemented as part of a corresponding resonant circuit, and the transmitter resonant circuit and the receiver resonant circuit are designed to have a common resonant frequency. Preferably, at least the transmitter resonant circuit, and more preferably both resonant circuits, are provided with an impedance tuning component, here implemented as a switchable or otherwise variable capacitance, in order to keep the resonant frequencies of the two circuits matched, and to keep the resonant frequency of the overall system within a desired target range under various operating conditions and varying degrees of coupling between the circuits. This impedance tuning may be a one-time adjustment on deployment of the system, or may advantageously be performed repeatedly during operation of the system. The external device (eyelid unit) preferably also includes resonance tracking (auto tuning) circuitry, which may be implemented as part of the RF driver or integrated as a function of the MCU, to adjust the driver frequency to the resonant frequency of the overall resonant system of the coupled resonant circuits.

Also illustrated here are two options for energy management on the ocular device side. In the event that power is supplied continuously, it may be sufficient to provide only "intermediate energy storage", which provides a smoothing and conditioning function on the output of the rectifier, but without significant energy storage capacity compared to the power rating of the device. Alternatively, the intermediate energy storage/conditioning components may charge an energy storage device, thereby allowing the ocular device to operate continuously even if only supplied intermittently with power by the external use (e.g., while blinking - see below).

In certain cases, data communication is needed between the ocular device and the external device, facilitating transfer of instructions to the ocular device, reporting of information sensed by the ocular device and/or contributing to the efficient operation of the power transfer between the two devices. FIG. 8B illustrates a first option for providing such communication. In this case, both the ocular device and the external device are provided with communication components, shown here as a transceiver and RF antenna, for wireless communication between the ocular device and the external device via transmission at a frequency that is typically, but not necessarily, different from the resonant frequency of the coupled resonant system. Typical examples of such communication components are transceivers configured to transmit and receive data according to standard NFC or BLE protocols. The communication components may employ dedicated antennae for this channel of communication. Alternatively, in some cases, the transmission and reception may be performed by superposition of the signals via the same antennae employed for the resonant coupling.

In some cases, the communication component of the external device employed for data transfer with the ocular device may also be used for communication with a network or mobile device. In other cases, due to differences in the required power and antenna design, it may be preferable to provide a separate communication component for communication with a network or mobile device, such as the BLE component illustrated here.

In all other respects, the structure and operation of the system of FIG. 8B is equivalent to that of FIG. 8A. It should again be appreciated that the drug delivery application illustrated here is merely an example, and that the system architecture is essentially generic to all of the potential applications listed above, both for contact lenses and implants.

Turning now to FIG. 9A, this shows a further example of a system which is essentially similar to that of FIG. 8B, but here provides data communication in one or both direction between the external device (eyelid unit) and the ocular device (eye unit) via modulation of the power transmission.

For data transfer from the external device to the ocular device, the external device MCU preferably switches the RF driver to convey data via modulation of an amplitude of power transfer to the ocular device. In one particularly simple implementation represented schematically in FIG. 9B, data can be conveyed by generating brief off/on pulses in the RF driver output, where different pulse durations or cycle times indicate a "0" or a "1". Clearly, this example is only one of an unlimited number of schemes for transferring data by amplitude modulation over the power signal. The modulation is then sensed by the ocular device, typically as variations in output voltage from the rectifier which can be sensed by the ocular device MCU, which decodes the transmitted date from the modulation.

For transmission of data from the ocular device to the external device, the MCU of the ocular device preferably encodes data by performing load modulation of the receiver resonant circuit, which causes a corresponding variation in the current of the transmitter resonant circuit, as illustrated in FIG. 9C. These variations are detected by envelope detection circuitry. The external device MCU then decodes the data from the sensed variations in the envelope of the transmitter resonant circuit power.

In all other respects, the structure and operation of the system of FIG. 9A is equivalent to that of FIG. 8B. In this case, the illustration shows intraocular pressure measurement as a non-limiting example of an application for which a capability of data transfer, continuously or at desired intervals, has particular value. It should be appreciated however that this application is merely an example, and that the system architecture is essentially generic to all of the potential applications listed above, both for contact lenses and implants.

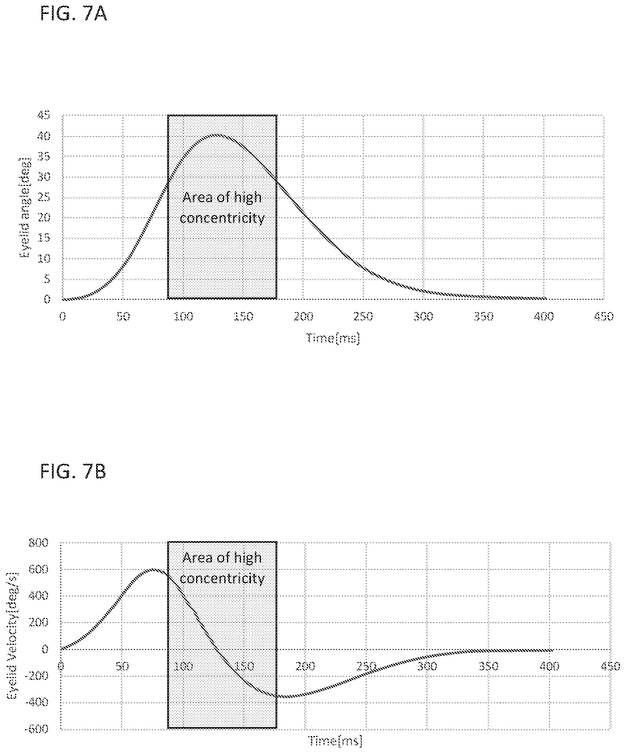

As mentioned earlier, in certain cases, it may be desired to perform power transfer and/or communications between the external device and the ocular device selectively during blinking, when the transmitter antenna and the receiver antenna are near optimum proximity and overlap. FIGS. 7A and 7B illustrate the angular displacement and angular velocity, respectively, of the upper eyelid during a typical blinking cycle. If the "area of high concentricity" is defined roughly as the 10 degrees closest to full closure, corresponding here to a deflection in excess of about 30 degrees, the duration of that period in this example is roughly 100 ms. As can be seen in FIG. 7B, the start and finish of this period corresponds approximately to the maxima (positive and negative respectively) in the velocity profile, enabling these points to be easily determined by an accelerometer. Alternatively, as discussed earlier, the load experienced by the transmitter circuit can be used to determine the current level of coupling between the transmitter circuit and the receiver circuit, and hence the current degree of overlap. (The oscillator can be actuated intermittently for sensing purposes even when energy or data transmission is not required.)

FIG. 9D illustrates schematically the operation of the RF driver of the external device as a function of time in a case where transmission of power and/or data is performed selectively on sensing of a blinking motion, and synchronously with that motion. The upper signal is the trigger signal, either from an accelerometer or derived from sensing of the load/coupling between the transmitter and receiver antennae. The lower signal represents the RF transmission that is triggered by the trigger signal. Optionally, the signal may also include variations caused by load switching in the ocular device to convey data to the external unit, as shown in the expanded view of the signal, all as described above.

It will be appreciated that the above descriptions are intended only to serve as examples, and that many other embodiments are possible within the scope of the present invention as defined in the appended claims.

## Claims

1. An external device (12) for communicating with an ocular device (10) deployed in or on an eyeball, the ocular device having a first communication subsystem, the external device comprising:
(a) an antenna (16) configured for attachment to an external skin surface of an eyelid of a
user or for mounting under the external skin surface of the eyelid of the user, so as to be separated from the eyeball by tissue of the eyelid and to move together with the eyelid, said antenna having a thickness of no more than 2 mm so as to allow folding of the skin of the eyelid over at least part of said antenna, and
(b) a second communication subsystem employing said antenna for transferring data wirelessly to and/or from the first communication subsystem.

2. A system for an eye of a user, the system comprising:
(a) an ocular device for deployment in or on an eyeball, said ocular device comprising a first communication subsystem;
(b) the external device of claim 1.

3. An external device for supplying energy to an ocular device deployed in or on an eyeball, the external device comprising:
(a) an electrically-actuated energy-transmitting component configured for attachment to an external skin surface of an eyelid of a user or for mounting under the external skin surface of the eyelid of the user, so as to be separated from the eyeball by tissue of the eyelid and to move together with the eyelid, said energy-transmitting component having a thickness of no more than 2 mm so as to allow folding of the skin of the eyelid over at least part of said energy-transmitting component, and
(b) driver circuitry, said driver circuitry being electrically connected to said energy-transmitting component for actuating said energy-transmitting component to transfer energy wirelessly to an energy receiver of the ocular device.

4. A system for an eye of a user, the system comprising:
(a) an ocular device for deployment in or on an eyeball, said ocular device comprising an energy receiver;
(b) the external device of claim 3.

5. The system of claim 4, wherein said energy-transmitting component comprises an antenna.

6. The system of claim 2 or claim 4, wherein at least part of said second communication subsystem or at least part of said driver circuitry is part of a unit configured for mounting on a region of the body of the user that does not move together with the eyelid.

7. The system of claim 2 or claim 4, wherein said second communication subsystem or said driver circuitry is integrated with said energy-transmitting component for mounting on or under the external skin surface of the eyelid of the user.

8. The system of claim 4, wherein said driver circuitry is further configured for encoding data for transmission to said ocular device via said energy-transmitting component.

9. The system of claim 2 or claim 4, wherein said external device further comprises a wireless communications interface for communicating with a communications network or with a mobile electronic device.

10. The system of claim 4, wherein said energy receiver comprises a receiver resonant circuit including a near-field receiver antenna, and wherein said energy-transmitting component comprises a near-field transmitter antenna which is part of a transmitter resonant circuit, said transmitter resonant circuit frequency matched to said receiver resonant circuit and coupled to said receiver resonant circuit, thereby forming a coupled resonant system, said driver circuitry being configured to actuate said transmitter resonant circuit so as to drive resonant oscillation of said coupled resonant system, and wherein said electronic circuitry of said ocular device recovers power from said receiver resonant circuit.

11. The system of claim 10, wherein said driver circuitry is configured to convey data via modulation of an amplitude of power transfer to said ocular device, and wherein said ocular device further comprises electronic circuitry associated with said receiver resonant circuit and configured to derive the data from said modulation.

12. The system of claim 10, wherein said ocular device further comprises electronic circuitry associated with said receiver resonant circuit and configured to convey data via load modulation of said receiver resonant circuit, and wherein said driver circuitry is configured to derive the data from said load modulation.

13. The system of claim 2 or claim 5, wherein said antenna comprises a conductive coil.

14. The system of claim 10, wherein said near-field receiver antenna and said near-field transmitter antenna are inductive antennae, or wherein said near-field receiver antenna and said near-field transmitter antenna are capacitive antennae.

15. The system of claim 10, wherein said near-field transmitter antenna is configured for attachment to an upper eyelid or wherein said near-field transmitter antenna is configured for attachment to a lower eyelid.

## Patentansprüche

1. Externe Vorrichtung (12) zum Kommunizieren mit einer Okularvorrichtung (10), die in oder auf einem Augapfel eingesetzt wird, wobei die Okularvorrichtung ein erstes Kommunikationsuntersystem aufweist, wobei die externe Vorrichtung umfasst:
(a) eine Antenne (16), die zum Anbringen auf einer externen Hautoberfläche eines Augenlids eines Benutzers oder zum Montieren unter der externen Hautoberfläche des Augenlids des Benutzers konfiguriert ist, um von dem Augapfel durch Gewebe des Augenlids getrennt zu sein und sich zusammen mit dem Augenlid zu bewegen, wobei die Antenne eine Dicke von höchstens 2 mm aufweist, um ein Falten der Haut des Augenlids über zumindest einem Teil der Antenne zu ermöglichen, und
(b) ein zweites Kommunikationsuntersystem, das die Antenne zum drahtlosen Übertragen von Daten an das und/oder von dem ersten Kommunikationsuntersystem einsetzt.

2. System für ein Auge eines Benutzers, wobei das System umfasst:
(a) eine Okularvorrichtung zum Einsetzen in oder auf einem Augapfel, wobei die Okularvorrichtung ein erstes Kommunikationsuntersystem umfasst;
(b) die externe Vorrichtung nach Anspruch 1.

3. Externe Vorrichtung zum Zuführen von Energie zu einer Okularvorrichtung, die in oder auf einem Augapfel eingesetzt wird, wobei die externe Vorrichtung umfasst:
(a) eine elektrisch betätigte Energieübertragungskomponente, die zum Anbringen auf einer externen Hautoberfläche eines Augenlids eines Benutzers oder zum Montieren unter der externen Hautoberfläche des Augenlids des Benutzers konfiguriert ist, um von dem Augapfel durch Gewebe des Augenlids getrennt zu sein und sich zusammen mit dem Augenlid zu bewegen, wobei die Energieübertragungskomponente eine Dicke von höchstens 2 mm aufweist, um ein Falten der Haut des Augenlids über zumindest einem Teil der Energieübertragungskomponente zu ermöglichen, und
(b) Antriebsschaltkreise, wobei die Antriebsschaltkreise elektrisch mit der Energieübertragungskomponente zum Betätigen der Energieübertragungskomponente verbunden ist, um Energie drahtlos an einen Energieempfänger der Okularvorrichtung zu übertragen.

4. System für ein Auge eines Benutzers, wobei das System umfasst:
(a) eine Okularvorrichtung zum Einsetzen in oder auf einem Augapfel, wobei die Okularvorrichtung einen Energieempfänger umfasst;
(b) die externe Vorrichtung nach Anspruch 3.

5. System nach Anspruch 4, wobei die Energieübertragungskomponente eine Antenne umfasst.

6. System nach Anspruch 2 oder Anspruch 4, wobei zumindest ein Teil des zweiten Kommunikationsuntersystems oder zumindest ein Teil der Antriebsschaltkreise Teil einer Einheit ist, die zum Montieren auf einem Bereich des Körpers des Benutzers konfiguriert ist, der sich nicht zusammen mit dem Augenlid bewegt.

7. System nach Anspruch 2 oder Anspruch 4, wobei das zweite Kommunikationsuntersystem oder die Antriebsschaltkreise mit der Energieübertragungskomponente zum Montieren auf oder unter der externen Hautfläche des Augenlids des Benutzers integriert sind.

8. System nach Anspruch 4, wobei die Antriebsschaltkreise weiterhin zum Codieren von Daten zur Übertragung an die Okularvorrichtung mittels der Energieübertragungskomponente konfiguriert sind.

9. System nach Anspruch 2 oder Anspruch 4, wobei die externe Vorrichtung weiterhin eine drahtlose Kommunikationsschnittstelle zum Kommunizieren mit einem Kommunikationsnetzwerk oder mit einer mobilen elektronischen Vorrichtung umfasst.

10. System nach Anspruch 4, wobei der Energieempfänger einen Empfängerresonanzkreis umfasst, der eine Nahfeldempfängerantenne beinhaltet, und wobei die Energieübertragungskomponente eine Nahfeldsenderantenne umfasst, die Teil eines Senderresonanzkreises ist, wobei die Senderresonanzkreisfrequenz auf den Empfängerresonanzkreis abgestimmt ist und an den Empfängerresonanzkreis gekoppelt ist, wodurch ein gekoppeltes Resonanzsystem gebildet wird, wobei die Antriebsschaltkreise dazu konfiguriert sind, den Senderresonanzkreis zu betätigen, um eine Resonanzoszillation des gekoppelten Resonanzsystems anzutreiben, und wobei die Elektronikschaltkreise der Okularvorrichtung Leistung von dem Empfängerresonanzkreis gewinnen.

11. System nach Anspruch 10, wobei die Antriebsschaltkreise dazu konfiguriert sind, Daten mittels Modulation einer Amplitude einer Leistungsübertragung an die Okularvorrichtung zu übermitteln, und wobei die Okularvorrichtung weiterhin Elektronikschaltkreise umfasst, die mit dem Empfängerresonanzkreis assoziiert sind und dazu konfiguriert sind, die Daten von der Modulation abzuleiten.

12. System nach Anspruch 10, wobei die Okularvorrichtung weiterhin Elektronikschaltkreise umfasst, die mit dem Empfängerresonanzkreis assoziiert sind und dazu konfiguriert sind, Daten mittels Lastmodulation des Empfängerresonanzkreises zu übermitteln, und wobei die Antriebsschaltkreise dazu konfiguriert sind, die Daten von der Lastmodulation abzuleiten.

13. System nach Anspruch 2 oder Anspruch 5, wobei die Antenne eine leitende Spule umfasst.

14. System nach Anspruch 10, wobei die Nahfeldempfängerantenne und die Nahfeldsenderantenne induktive Antennen sind oder wobei die Nahfeldempfängerantenne und die Nahfeldsenderantenne kapazitive Antennen sind.

15. System nach Anspruch 10, wobei die Nahfeldsenderantenne zum Anbringen auf einem oberen Augenlid konfiguriert ist oder wobei die Nahfeldsenderantenne zum Anbringen auf einem unteren Augenlid konfiguriert ist.

## Revendications

1. Dispositif externe (12) pour communiquer avec un dispositif oculaire (10) déployé dans ou sur un globe oculaire, le dispositif oculaire présentant un premier sous-système de communication, le dispositif externe comprenant :
a) une antenne (16) configurée pour être fixée sur une surface de peau externe d'une paupière d'un utilisateur ou pour être montée sous la surface de peau externe de la paupière de l'utilisateur, de manière à être séparée du globe oculaire par le tissu de la paupière et à se déplacer avec la paupière, ladite antenne présentant une épaisseur non supérieure à 2 mm de manière à permettre le pliage de la peau de la paupière sur au moins une partie de ladite antenne, et
b) un second sous-système de communication utilisant ladite antenne pour transférer sans fil des données vers et/ou depuis le premier sous-système de communication.

2. Système pour un œil d'un utilisateur, le système comprenant :
a) un dispositif oculaire destiné à être déployé dans ou sur un globe oculaire, ledit dispositif oculaire comprenant un premier sous-système de communication ;
b) le dispositif externe selon la revendication 1.

3. Dispositif externe pour fournir de l'énergie à un dispositif oculaire déployé dans ou sur un globe oculaire, le dispositif externe comprenant :
a) un composant de transmission d'énergie actionné électriquement configuré pour être fixé sur une surface de peau externe d'une paupière d'un utilisateur ou pour être monté sous la surface de peau externe de la paupière de l'utilisateur, de manière à être séparé du globe oculaire par le tissu de la paupière et à se déplacer avec la paupière, ledit composant de transmission d'énergie présentant une épaisseur non supérieure à 2 mm de manière à permettre le pliage de la peau de la paupière sur au moins une partie dudit composant de transmission d'énergie, et
b) un ensemble de circuits d'attaque, ledit ensemble de circuits d'attaque étant électriquement connecté audit composant de transmission d'énergie pour actionner ledit composant de transmission d'énergie afin de transférer sans fil de l'énergie à un récepteur d'énergie du dispositif oculaire.

4. Système pour un œil d'un utilisateur, le système comprenant :
a) un dispositif oculaire destiné à être déployé dans ou sur un globe oculaire, ledit dispositif oculaire comprenant un récepteur d'énergie ;
b) le dispositif externe selon la revendication 3.

5. Système selon la revendication 4, dans lequel ledit composant de transmission d'énergie comprend une antenne.

6. Système selon la revendication 2 ou la revendication 4, dans lequel au moins une partie dudit second sous-système de communication ou au moins une partie dudit ensemble de circuits d'attaque fait partie d'une unité configurée pour être montée sur une zone du corps de l'utilisateur qui ne se déplace pas avec la paupière.

7. Système selon la revendication 2 ou la revendication 4, dans lequel ledit second sous-système de communication ou ledit ensemble de circuits d'attaque est intégré audit composant de transmission d'énergie pour un montage sur ou sous la surface de peau externe de la paupière de l'utilisateur.

8. Système selon la revendication 4, dans lequel ledit ensemble de circuits de commande est en outre configuré pour coder des données à des fins de transmission audit dispositif oculaire par l'intermédiaire dudit composant de transmission d'énergie.

9. Système selon la revendication 2 ou la revendication 4, dans lequel ledit dispositif externe comprend en outre une interface de communication sans fil pour communiquer avec un réseau de communication ou avec un dispositif électronique mobile.

10. Système selon la revendication 4, dans lequel ledit récepteur d'énergie comprend un circuit résonnant récepteur incluant une antenne réceptrice en champ proche, et dans lequel ledit composant de transmission d'énergie comprend une antenne émettrice en champ proche qui fait partie d'un circuit résonnant émetteur, ledit circuit résonnant émetteur étant accordé en fréquence sur ledit circuit résonnant récepteur et couplé audit circuit résonnant récepteur, formant ainsi un système résonnant couplé, ledit ensemble de circuits d'attaque étant configuré pour actionner ledit circuit résonnant émetteur de manière à entraîner une oscillation résonnante dudit système résonnant couplé, et dans lequel ledit ensemble de circuits électroniques dudit dispositif oculaire récupère l'énergie dudit circuit résonnant récepteur.

11. Système selon la revendication 10, dans lequel ledit ensemble de circuits d'attaque est configuré pour transmettre des données par modulation d'une amplitude de transfert de puissance vers ledit dispositif oculaire, et dans lequel ledit dispositif oculaire comprend en outre un ensemble de circuits électroniques associé audit circuit résonnant récepteur et configurés pour dériver les données à partir de ladite modulation.

12. Système selon la revendication 10, dans lequel ledit dispositif oculaire comprend en outre un ensemble de circuits électroniques associé audit circuit résonnant récepteur et configuré pour transmettre des données par modulation de charge dudit circuit résonnant récepteur, et dans lequel ledit ensemble de circuits d'attaque est configuré pour dériver les données à partir de ladite modulation de charge.

13. Système selon la revendication 2 ou la revendication 5, dans lequel ladite antenne comprend une bobine conductrice.

14. Système selon la revendication 10, dans lequel ladite antenne réceptrice en champ proche et ladite antenne émettrice en champ proche sont des antennes inductives, ou dans lequel ladite antenne réceptrice en champ proche et ladite antenne émettrice en champ proche sont des antennes capacitives.

15. Système selon la revendication 10, dans lequel ladite antenne émettrice en champ proche est configurée pour être fixée à une paupière supérieure ou dans lequel ladite antenne émettrice en champ proche est configurée pour être fixée à une paupière inférieure.
